(19) Europäisches Patentamt
European Patent Office
Office européen des brevets

(11) EP 3 926 641 A1

(12) EUROPEAN PATENT APPLICATION

(43) Date of publication:
**22.12.2021 Bulletin 2021/51**

(21) Application number: **20465536.9**

(22) Date of filing: **16.06.2020**

(51) Int Cl.:
***G16H 40/40*** *(2018.01)*
***G16H 40/60*** *(2018.01)*
***G16H 20/17*** *(2018.01)*
***A61B 5/00*** *(2006.01)*
***G08B 21/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fresenius Medical Care Deutschland GmbH**
**61352 Bad Homburg (DE)**

(72) Inventors:
- **NOVAC, Nicolae**
  **Bucharest (RO)**
- **TRAUNER, Karin Helga**
  **97422 Schweinfurt (DE)**

(74) Representative: **Schwarz, Claudia**
**Schwarz + Kollegen**
**Patentanwälte**
**Hermann-Schmid-Straße 10**
**80336 München (DE)**

# (54) TIME-CONTROLLED ALARM HANDLING FOR A MEDICAL DIALYSIS DEVICE

(57) In one aspect the invention relates to a computer-implemented method for determining a set of alarm messages (am) for operating a medical device (MD). The method comprises:
- Receiving (S1) measurement data (md) with a temporal indication (ti) from a set of sensors and/or data providers;
- Aggregating (S2) the received measurement data (md);
- Processing (S3) the aggregated data (ag) by applying a temporal alarm significance algorithm (TASA) for providing a result dataset (rd) with a determined set of alarm messages (am) and a calculated execution time, defining when the determined set of alarm messages (am) are to be executed.

FIG. 8

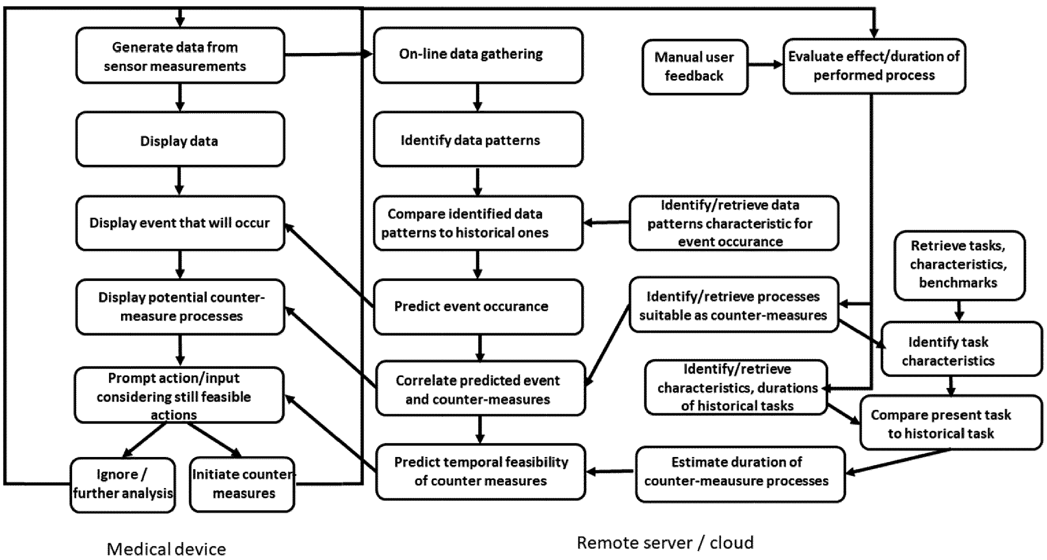


EP 3 926 641 A1

## Description

The present invention relates to alarm handling for medical devices, in particular dialysis devices and related treatment devices.

In general, medical treatment devices require a high degree of reliability and accuracy, and secure operation needs to be safeguarded. Appropriate alarm handling is therefore key for the patient's health.

In case a treatment device or related equipment (such as disposables) or the patient's health condition is/are in a problematic state(s), which require respective intervention, modern medical systems typically are equipped with alarm modules for communicating such possible alarm states as early as possible so that respective countermeasures may be initiated. These countermeasures serve to prevent critical states during medical treatment and to provide operating instructions for supporting the medical personnel in executing the countermeasures.

In the state of the art, it is known to provide the medical device or its components or patient-related healthcare devices with alarm modules for emitting an alarm signal, in case the device or any of its components is detected to be in an erroneous state or even in a problematic state. For safeguarding that every potentially dangerous state is communicated as early as possible, in state-of-the-art systems, every alarm signal is usually provided as soon as it has been issued. This, however, leads to situations, where alarms are provided although the situation or condition is not yet critical for the patient. For example, the volume of the dialysate or concentrate bag is measured continuously. It is predicted to be empty in 1 hour. In order to provide sufficient security, the alarm signal is controlled to be issued well before becoming empty in order to provide sufficient time for the staff to change the bag and to replace it with a new bag. Therefore, the *"empty bag"* alarm signal may be issued too early for scenarios in which the appropriate countermeasure (bag change) may be executed within e.g. 5 minutes (because the new bag is well prepared and accessible at the device and the *"change bag"* action may only require 5 minutes). This may lead, in turn, to a situation in which the alarm signal is issued 50 minutes too early, so that the nurse, being very busy, simply clicks the alarm off or away. Typically, the alarm will be reiterated for a few times, and every new alarm may be replied with a *"click away"* action by the nurse. In case this is done repeatedly and too often, finally, a critical situation may arise, so that the bag actually will be empty without having initiated the necessary countermeasure. In this scenario, it is helpful to provide the means to generate the alarm message in a manner which is adapted to the current user setting and the duration of the countermeasure. In this particular example, it would be helpful to provide the alarm message only as needed, taking into account the actual duration of the countermeasure (bag change, attendance of medical personal) e.g. 15 minutes before the predicted total draining.

In another example, a patient is known or expected to experience health problems during a medical dialysis treatment. In state-of-the-art systems, the patient's vital data are monitored and as soon as these measured vital data are outside a predefined normal interval, the alarm will be triggered. However, it may turn out that the appropriate countermeasures (e.g. for kidney problems) may require a longer period of preparation. Then, although the alarm signal was issued as early as possible, based on measurement data, the remaining time may not be sufficient to initiate the appropriate steps (e.g. organizing an extracorporeal blood-circulation machine). In this case, it is helpful to provide means to trigger an alarm message earlier so that enough time will be left to execute the countermeasure.

Another example refers to the fact that the patient's vital data are monitored during treatment. In case one specific data item (e.g. temperature) runs out of the predefined "normal" range, the alarm signal will be prompted. However, the very vital parameter may be subject to temporal variations and exceeding or shortfalls of thresholds will not instantaneously lead to a problematic situation for the patient. In contrast, the parameter value may change again to return to within the normal range and treatment may continue without intervention. In such case, it would be helpful to not directly issue the alarm signal as soon as it has been issued, but to monitor the same over time and "wait", whether or not the vital-data parameter will become normal again. In the latter case, there is no need to trigger an alarm as the parameter value has recovered over time.

Therefore, an object of the present invention is to provide a method, an alarm handler, a medical device and a computer program according to the accompanying claims, in particular electronic means that allow to consider and process temporal data for the alarm message generation. Further, alarm handling during medical treatment should become safer and alarm messages should be provided more reliably. Unnecessary alarm signals and alarms which are not time-related to the predicted durations of respective countermeasures should be avoided. The accuracy of alarm message should be enhanced.

According to a first aspect of this disclosure, this object is solved by a computer-implemented method for determining a set of alarm messages, which may be appended or may comprise appropriate mitigation instructions or countermeasures, for operating a medical device (e.g. a dialysis device) or medical equipment (e.g. disposables), comprising the steps of:

- Receiving measurement data with a temporal indication (e.g. time stamp) from a set of sensors (e.g. temperature sensor for monitoring one of patient's vital data, pressure sensor of a dialysis device etc.) and/or data providers (e.g. external, network-associated databases, e.g. for providing replacement data for a mechanical part of the treatment machine etc.); wherein the measurement data may include medical treatment data from the treatment device or from a patient's device (smart watch etc.) or from other equipment or from an equipment provider, in particular tool or product lifetime or original alarm signals from a device, as well as data of the prediction module for predicting alarms; the prediction module itself may be based on alarm signals of the components of the medical device or on measurement data;

- Aggregating the received measurement data;
- Processing the aggregated data by applying a temporal alarm significance algorithm for providing a result dataset with a determined set of alarm messages and a calculated execution time, defining when the determined set of alarm messages are to executed or output or provided, e.g. on a user interface related to the medical treatment device.

This method has several advantages. Due to applying the temporal alarm significance algorithm, temporal developments of signals and measurement values may be processed for the determination of the alarm message and provisioning of the same. This has the technical effect and advantage that the provision of the alarm message may be done more reliable as only those alarm messages are generated which convey the need for direct intervention. Further, the determination of alarm messages may be adapted to the present user setting and scenario and, in particular, to the duration of possible countermeasures. For example, in case the countermeasures are predicted to take longer, then, the respective alarm message will be provided earlier and vice versa, if the countermeasures are estimated to be faster, then, the alarm message may be provided later but still in due time before a critical situation will arise. Thus, unnecessary (for that moment being) or even wrong alarm messages or alarm messages which are issued too early or to late may be avoided. Thus, the user, e.g. the nurse or the medical personnel, or the patient, may better rely on the need to execute a countermeasure or to intervene if an alarm message was provided. Notifications directed to the user may be issued earlier, if needed, or later, if possible, in the present application case setting. Finally, security of medical treatment may be improved due to better and more specifically adapted alarm and error message handling.

Preferably, the determination of the set of alarm messages may be based on estimated data or predicted data. In this embodiment, the currently detected measurement data may serve as a "first source" of data which are fed to a "second source", namely to a prediction module, first, before they are used for the determination of the alarm signal. The prediction module may comprise a set of algorithms, e.g. an artificial neural network, which has been trained with historical measurement-data patterns and assigned probabilities for critical event occurrences in order to be able to later on automatically predict a probability for a critical event occurrence for a new (unobserved) measurement-data pattern (set of different types of measurement data). The prediction module serves as a forecast system for estimating probabilities for problematic event occurrences, based on measurement data. Based on these estimated or predicted event occurrences another set of algorithms may be applied to determine and generate an appropriate countermeasure and/or alarm message, which

1. is feasible and available (including the preparation time for initiating the countermeasure) within the time left until the event is estimated to occur and
2. needs to be executed within a pre-definable time interval in order to prevent, or at least mitigate, the estimated event to occur beforehand.

In a preferred embodiment, the temporal alarm significance algorithm may comprise:

- Based on the aggregated data: Predicting at least one (problematic) event occurrence with its time or temporal indication, in particular, at what time the event is predicted (this may be based on historic data and/or may be determined by a trained neural network and/or a machine learning algorithm;
- Determining at least one countermeasure for the predicted event occurrence and its estimated availability (time-based, e.g. after 10.00 p.m., or event-based, e.g. a predefined set of conditions is fulfilled, e.g. stock replenishment of disposables which is electronically controlled) and duration (how long the execution of the countermeasure will last);
- Determining the set of alarm messages with countermeasures which have been analyzed to be available and temporally feasible and necessary to prevent or mitigate the predicted event prior to its predicted occurrence by taking into account the estimated availability and duration of the countermeasure in particular in relation to the current system time.

This has the technical advantage that potentially problematic issues or events may be determined which are due to a combination of several parameters, which are provided by the aggregated data. Particularly, a dangerous setting may arise if measurement value A is below threshold 1 and if measurement value B is above threshold 2 and the required new disposable is within a range of 200 meters. Further, the prediction is based on real historical data and thus the reliability of the prediction may be improved.

In another embodiment, analyzing the availability of a countermeasure may be based on device-external data and it may in particular be based on a clinical infrastructure parameter (representing how easy and fast certain locations may be reached), location-based parameters of the patient and/or of required devices and/or staff for executing the countermeasures, user-experience parameters (representing how experienced/efficient the nurse is in executing the countermeasure), allocations of disposables and/or equipment for countermeasure execution. This improves the quality of prediction and the adaption to the current setting.

In another preferred embodiment, the method may comprise:

- Providing the determined result dataset and/or set of alarm messages on an output device at the determined execution time.

It has to be noted, that the point of time, at which the alarm message is generated (calculated), may differ from its execution time, i.e. the point of time at which the generated alarm message is "executed", which means, that the alarm message is prepared to be provided or issued on a user interface. In general, the output or provisioning is scheduled in accordance with temporal conditions, particularly with respect to the duration of the countermeasures. Furthermore, the generation of the alarm message may be executed on another entity as the provision of the same. For example, the alarm message may be generated on a network-accessible server and may be output on a user interface of the medical treatment device or on a user interface of a mobile device of medical treatment personnel. This makes the system very flexible.

In a further preferred embodiment, the measurement data may be received from a medical treatment device (e.g. dialysis machine), a patient's device (e.g. heart-rate sensor in a smart device) and/or equipment related to the patient (e.g. disposable), and/or from a data provider (e.g. the manufacturer of the medical treatment device or from a repository of the medical site etc.). The measurement data may cumulatively or alternatively be received from a prediction module. Generally, the measurement data may stem from different data sources. The measurement data may be discrete or continuous data in different or identical formats. Preferably, the measurement data are acquired from a huge variety of different data sources, relevant for the medical treatment. This helps to improve the quality of the predictions.

In another preferred embodiment, the measurement data may comprise original alarm signals, issued by a medical treatment device and wherein the determined set of alarm messages is a subset (in case original alarm signals are postponed or suspended from being executed later) or a superset of the original alarm signals (in case the original alarm signals are analyzed as being urgent to be executed and output together with other alarm signals or messages, inter alia together with operating instructions for handling the alarm). Thus, the alarm generators of components of the treatment device are modified such as their output signal may be taken as input of the method described herein. In some cases, the original alarm signal is analyzed to be handled as alarm message and may be prepared for output directly or in a later phase. In other cases, the original alarm signal is analyzed not to be handled as alarm message, e.g. in case further monitoring of the underlying measurement signal is indicated and triggered and in case the signal then is determined to show a development over time that represents a normal condition again. So, the original alarm signal would have been superfluous as the respective measurement parameter has returned to normal value again.

In another preferred embodiment, the countermeasures may be determined by executing a countermeasure algorithm, which may be based on a first machine-learning algorithm which has been trained in a supervised or semi-supervised manner with training data of historical problematic events and associated countermeasures. The first machine-learning algorithm may be based on a first trained model.

In still another preferred embodiment, the execution time for the determined set of alarm messages may be calculated by processing estimated process durations of countermeasures. This has the technical advantage that the method and system may be adapted more specifically to the particular field of application. For example, if distances are large in a big infrastructure (hospital) so that the staff or medical personnel needs a long time to get to the patient's site for initiating several countermeasures, the time for executing these countermeasures will be longer compared to the time to be spent in a small entity where everyone and everything is currently at hand and on place. Differences of this kind are considered in the solution presented herein when providing the result dataset with the countermeasures.

In still another preferred embodiment, process durations for execution of the countermeasures may be estimated by accessing a storage with a countermeasure data structure in which each countermeasure is assigned a unique identifier (which serves for identifying the respective countermeasure electronically) and corresponding characteristic properties, including process duration so that process durations may be determined for processes which have similar characteristic properties. In simple words, the storage may include a kind of table (in simplified terms), storing historical events with historical appropriate countermeasures, which have been analyzed to be appropriate for mitigating the problematic or dangerous event. Thus, this data structure may be accessed in order to find those countermeasures which have a high matching score (for example a matching score above a preset threshold). This feature makes it possible to learn from past events and its solutions by means of applying the appropriate countermeasures.

In still another embodiment which has been proven as advantageous, the countermeasures may include meta data, comprising operating instructions on a step-by-step basis. The meta data may thus include a temporal execution of countermeasure steps, potentially executed on different entities and/or devices. This helps the nurse to operate on the device and to execute the correct sequence of countermeasures steps in order to eliminate the problematic event. In particular, this feature is helpful in case several actions need to be taken in order to eliminate a complex problem.

In still another embodiment of the present situation, the determined set of alarm messages may comprise a reliability indicator and/or an accuracy score of predicted data. In a preferred embodiment, the accuracy score and that reliability indicator is/are determined for both, the problematic events and corresponding countermeasures. With this indication the user gets more information on how precise the determined estimation is and how thoroughly he/she may rely on the calculations. In case it turns out that that accuracy score is below a preset threshold and the situation is not urgent (in that the health status of the patient is not jeopardized), the algorithm (the system) may e.g. decide to wait whether or not the underlying medical situation relaxes and parameters, which signified the problematic event, may return to normal so that the mitigation procedure needs not to be executed. The "waiting" for an improvement of the underlying measurement data (change for the better) may be indicated on a user interface for user verification, so that the user will be notified of the system's waiting decision.

In the following, the solution of this disclosure will be described with respect to the claimed apparatus. Features, advantages or alternative embodiments mentioned with respect to the method can also be assigned to the other claimed or described subject matters (e.g. apparatus, the computer program or a computer program product) and vice versa. In other words, the subject matter of the apparatus (alarm handler) can also be improved or further developed with features described or claimed in the context of the method. In this case, the functional features of the method are embodied by structural units of the system, configured to dedicatedly execute this function and vice versa, respectively. Generally, in computer science at least from a computational point of view, a software implementation and a corresponding hardware implementation are equivalent. Thus, for example, a method step for "storing" or "aggregating" data may be performed with a "storage unit" and respective instructions to write data into the storage. For the sake of avoiding redundancy, these embodiments are not explicitly described again for the apparatus, because they have been described already in relation to the method.

In another aspect the present invention relates to an alarm handler, which may be implemented as electronic module, for determining a set of alarm messages. The alarm messages may include mitigation instructions with countermeasures to be taken by a personell on site or by initiating procedures upon receiving a verification signal or even automatically. The alarm handler is preferably provided for operating a medical device or equipment or disposable, configured to perform a method as described above, comprising:

- A measurement interface which is adapted for receiving measurement data with a temporal indication from a set of sensors and/or data providers;
- A storage for aggregating the received measurement data; and
- A processing unit for processing the aggregated data by applying a temporal alarm significance algorithm (TASA) for providing a result dataset with a determined set of alarm messages and a calculated execution time, defining when the determined set of alarm messages are to executed or issued/ provided on a user interface coupled to or implemented on a device. The TASA algorithm may further call or execute or comprise a countermeasure algorithm (CMA).

In still another aspect of the present invention refers to a medical device with an output interface and further comprising an alarm handler as described above.

In another aspect of the present invention relates to a computer program product comprising a computer program, the computer program being loadable into a memory unit of a processing unit, including program code sections to make the processing unit execute the method for determining a set of alarm messages as described above, when the computer program is executed in said processing unit.

In another aspect the invention relates to a computer-readable medium, on which program code sections of a computer program are stored or saved, said program code sections being loadable into and/or executable in a processing unit to make the processing unit execute the method for determining a set of alarm messages as described above, when the program code sections are executed in the processing unit.

The realization of the invention by a computer program product and/or a computer-readable medium has the advantage that already existing medical treatment devices and related servers and/or IoT (internet of things) and/or clients can be easily adopted by software updates in order to function as proposed by the invention.

In the following, a definition of terms used within this application is given.

Generally, in the solution described herein, temporal data are highly relevant and are processed for providing the set of alarm messages. In particular, at least three types of time or temporal indicators [as e.g. time stamps] are used:

1. the temporal indication of each measurement data,
2. the estimated duration of the determined countermeasure, representing the time, how long the countermeasure may take to be prepared and executed; this may also include a time interval, which is necessary to prepare the countermeasure, for instance to get the physician on spot (to the device),
3. the predicted event occurrence: this time data may be provided in an absolute indication (e.g. "at 8:15 on DATE") or as relative indication, relative to system time (e.g. "in 20 minutes");

4. the actual system time.

This feature makes it possible to provide only a subset of original alarm signals, namely those, which are analyzed to have countermeasures which are feasible before predicted event occurrence). With this, it is possible to provide the alarm messages in a more usable and useful format for practical application, which renders the machine or device operation safer and more efficient (less distraction due to unnecessary alarm notifications).

The term "execution" of the set of alarm messages is to be construed in the sense of providing the alarm messages, e.g. on a user interface, or output of the alarm message (e.g. in an acoustical format).

The computer for executing the method for determining a set of alarm messages as described above and in particular for executing the temporal alarm significance algorithm and/or the countermeasure algorithm, mentioned above, may be a personal computer, a server, a router, a peer device, a common network node, and/or a workstation in a computer network and may include a processing unit, a system memory, and a system bus that couples various system components including the storage means to the processing unit. The computer may be implemented in or as a data processing system (also: computer-implemented system). Said data processing system may thus comprise a computing device or a cluster thereof. The data processing system comprises a processing unit and storage means, which are in data communication with one another. The processing unit may consist of or comprise a CPU and/or a GPU and comprises several modules configured to perform the steps of the method of the present invention. The central processing unit may comprise hardware and software components, the latter also in the form as embedded software and/or algorithms. The CPU may comprise or may be provided as a microprocessor, a field programmable gate array (an acronym is "FPGA") or an application specific integrated circuit (an acronym is "ASIC"). The CPU may serve to execute different internal functions of computing entities of the medical device. In addition to this, the CPU may further be designed to generate a set of alarm messages and to trigger the detection of measurements and/or treatment and/or device related parameters and to issue the result dataset and to prepare it to be displayed on the display unit, i.e. to calculate a graphical representation of the result data (optionally including graphical elements) for being displayed on e.g. a monitor of the treatment device or a mobile device.

A computer program may be stored on a suitable medium, such as an optical storage medium or a solid-state medium, supplied together with or as part of other hardware, but may also be distributed in other forms, such as via the Internet or other wired or wireless telecommunication systems. The computer program may be or may comprise an executable for execution on a processor or processing unit. The computer program may be provided in a distributed manner, and may, thus, be distributed between several computing entities, such as processors. For example, the computer program may be distributed (stored) on different computing entities, like a set of clients (acting as IoT devices, like an IoT-medical treatment device) and a server, so that a portion of the computer program resides on a first computing entity and another portion resides on a second entity. The computer program may comprise program modules, which may include routines, programs, objects, components, data structures, etc. to instruct the computer to perform at least one task by data processing, in particular of abstract data types.

The storage means may comprise volatile primary memory (e.g. a RAM, a DRAM, a SRAM, a CPU cache memory or the like) and/or non-volatile primary memory (e.g. a ROM, a PROM, an EPROM or the like). In particular, the volatile primary memory may consist of a RAM. For instance, the volatile primary memory temporarily holds program files for execution by the processing unit and related data and the non-volatile primary memory may contain bootstrap code for the operating system of the data processing system. The storage means may further comprise additional memory, which may store the operating system and/or the instructions of the algorithms used to carry out the method of the present invention, in particular the TASA and/or the CMA algorithm and/or machine-learning algorithms. Moreover, the additional memory may store a computer program product comprising instructions, which, when the computer program product is executed by the data processing system, cause the data processing system to carry out the method according to the present invention.

Generally, the components of the system, in particular the additional memory, the primary memories, and the processing unit need not be physically housed within the same housing and may instead be spatially separated from one another. In particular, the additional memory, the primary memories, and the processing unit may be spatially separated from one another and may exchange data with one another via wired and/or wireless media.

The invention may use a set of algorithms. An algorithm is, in particular, a collection, e.g. a sequence, of instructions for processing input information to obtain output information. The instructions of an algorithm may be implemented in a computer and executed by a processor, e.g. of the data processing system according to the present invention. In particular, the input information of the algorithm is encoded in input data (here: measurement data) that may be accessed by the processor carrying out the algorithm. In particular, the processor processes the input data according to the instructions of the algorithm to generate output information, which, typically, is encoded in output data. According to the present invention, in particular, an algorithm processes data when the algorithm comprises instructions, which, when executed by a processor, cause said processor to process these data.

In particular, a machine-learning algorithm comprises instructions for processing input information to obtain output information and at least some of these instructions are set by using a set of training data and a training algorithm. The machine learning algorithm may comprise an Artificial Neural Network (ANN), a decision tree, a random forest, a support vector machine (SVM), or the like. For example, the machine learning algorithm may be a convolutional neural network (CNN) and/or a deep neural network (DNN), such as AlexNet. In particular, an ANN is an algorithm that maps an ANN input into an ANN output and that comprises a plurality of interconnected nodes, each node mapping an input into an output by means of an activation function, which may be node-dependent. In particular, the activation function of a node may depend on a bias and on one or more weights weighting the data items of the input of said node. The values of the weights of the ANN may be obtained by training the ANN by means of at least a training set and a training algorithm. According to an exemplary training algorithm, the values of the weights are iteratively adjusted so as to minimize the value of a cost function which, typically, depends on the weights of the ANN, the ANN inputs, the ANN outputs, and/or the biases.

The data processing or computer-implemented system may comprise an input/output (I/O) interface which allows the data processing system to communicate with input/output devices (e.g. displays, keyboards, touchscreens, printers, mice, cameras, or the like). The data processing system may further comprise a network interface controller (NIC) configured to connect the data processing system with a suitable network. According to the present invention, a suitable network may for instance be an intranet, the internet or a cellular network. The computer-implemented system may operate in a networked environment which defines physical connections to one or more remote computers. The remote computer may be another computer, as defined above. The physical connections include a local area network (LAN) and a wide area network (WAN), an intranet and the Internet.

The medical device is a technical apparatus for medical purposes or medical treatment. It comprises medical units and computing and/or electronic units which mainly serve to control and operate the medical device. In a preferred embodiment, the medial device is a blood treatment device, in particular a dialysis device. The medical device may also be an apparatus used for peritoneal dialysis or a plasmapheresis device. Dialysis is commonly used to replace kidney function by removing waste toxins and excess water. During treatment and thus during operation with the medical device, correct operation needs to be assured. Thus, a variety of different alarm signals may be issued in order to verify certain state of the machine/device or to apply countermeasures.

The set of sensors comprises at least one sensor for measuring technical, physical and/or physiological data in relation to the treatment of a patient with the medical device. The sensors may be implemented directly on the medical device or on related devices, such as a smart device, for example a smartwatch, a patient wristband, a heart-rate monitoring device or the like. The set usually comprises a plurality of different types of sensors. The sensors may be temperature sensors, flow sensors, and/or pressure sensors, and the like. The sensor signals, however, may not only be acquired by means of the sensors, but also be acquired by means of reading data from memory, storing machining parameters, like e.g. technical state information as machine/device type, operating duration, installed software, and/or energy consumption, etc.

The prescription module may be implemented on a central processing unit or graphical processing unit. The prescription module may implement a set of algorithms for execution. The set of algorithms may serve to predict permanent or temporary failures, losses, impairments, or breakdowns of the medical device or parts thereof or other components related to the medical treatment or of necessary equipment for medical treatment, like e.g. disposables, which are referred to as "(problematic) event". For example, the prediction module may estimate, e.g. event occurrences on historical data by modelling the technical functions related to normal and faulty operation of the device or treatment process. For example, the equipment manufacturer of disposables may issue a notification that a disposable needs to be changed again after a pre-defined set of working hours. Thus, a notification may be provided, issued from an electronic module related to the disposable and/or to the medical device that the particular disposable needs to be changed within, for example, one hour. This "one-hour notification" may also serve as measurement data and will be processed by the temporal alarm significance algorithm. In contrast to state-of-the-art systems, however, this "one-hour notification" will no longer be instantaneously provided directly on a user interface, but will be processed, so that for example it may be provided in a later stage - if appropriate (e.g. if a new replacement disposable is right at hand and available and staff for replacement action is also available) - or may be provided in a processed manner, e.g. including time-related data.

The output interface or output device may be a display unit and may be provided as a monitor, integrated into the medical device or provided as separate unit in data exchange (e.g. smart device, mobile device). It may, in particular, be a touch-sensitive display. The output interface serves to provide the result dataset.

Alternatively, or in conjunction with the above, the method may further comprise the step of:

- generating a virtual representation of at least a part of the result dataset, in particular of the countermeasures to be executed.

The virtual representation of the countermeasures may be stored and provide an automatically generated documentation of the countermeasure procedure carried out by the computing entity. Alternatively, or in conjunction with the above, the virtual representation may be used to automatically validate and, if needed, correct the procedure. Moreover, by using the virtual representation, the system may automatically infer the procedure to be carried out and in addition the user may be led towards a sequence of steps to be taken.

The alarm message is a digital message which is calculated and generated on the CPU, based on the provided and received alarm signals and/or measurement data, potentially being provided by the components of the medical device or by components related to the medical treatment. The alarm message may be provided as a part of a result dataset on a user interface or on another type of output device (acoustic output etc.). An alarm message may for example relate to *"concentrate bag needs to be changed at the latest in 10 minutes"* or *"urgent intervention is necessary due to measurement of patients vitals"* etc.

The measurement data may comprise at least two different types of parameters:

1. technical parameters of the medical device or of related treatment devices and
2. medical or physiological parameters, relating to the medical treatment with the device and/or to health data of the patient during treatment.

The technical parameters may e.g. relate to a hemodialysis, hemodiafiltration or peritoneal dialysis apparatus and may comprise data representing the type of the device, year of construction, serial number, software versions installed, available treatment options, blood pumping rate, a rate or temperature of dialysis fluid or dialysate, transmembrane pressure (e.g. TMP). The TMP usually is decisive for a particular ultrafiltration rate and is usually regulated/controlled by the corresponding control of the extracorporeal blood circulation and dialysis or dialysis substitution fluid flows, i.e. blood pumps, dialysis fluid pump, if necessary ultrafiltration pump and substitute(nt) pump(s). The technical parameter may further comprise a total or overall running time of the machine or medical device. This refers to the total running time of the machine, which is documented. If there is a correlation between frequently occurring alarms and a typical total running time, this may indicate a problem on the machine side, e.g. wear. The technical parameter may further comprise used disposables, like filters, tube sets, solvent compositions, etc. The technical parameter may further comprise temperature, measured at important locations within the medical device, like e.g. at the central processing unit, at the pumps, the sensor casing temperature. The sensor casing temperature may be correlated the outside temperature which may be provided by means of external temperatures sensors. The technical parameter may further comprise the state of the electric supply, like voltage value and power consumption for calculating further data, inlet volume in peritoneal dialysis device, dwell time of the dialysis fluid in peritoneal dialysis device, effluent volume in peritoneal dialysis device (coupled to the inlet volume), temperature of the latter. The temperature of the effluent volume may indicate a wrong or bad temperature of the patient, i. e. being too high or too low with respect to pre-defined values. The effluent volume in a peritoneal dialysis device may be measured for determining the ultrafiltration volume. This parameter relates to the medical background that the ultrafiltrate passes over into the peritoneum and the respective volume adds to the dialysate volume. If the dialysate remains in the peritoneum for a too long time, the dialysate may be resorbed via the peritoneum, which has the consequence that the patient will not be dehydrated as intended but may even receive a fluid bolus.

The medical parameters may also relate to measured values during treatment, e.g. physiological and/or medical values of the patient at the point of time, at which the alarm signal has been issued or was provided, like e.g. an accumulated ultrafiltration volume, blood pressure, a Kt/V value. Kt/V is a number used to quantify hemodialysis and peritoneal dialysis treatment adequacy, wherein K refers to dialyzer clearance of urea, t is dialysis time and V is the volume of distribution of urea, approximately equal to patient's total body water. In the context of hemodialysis, Kt/V is a pseudo-dimensionless number and is dependent on the pre- and post-dialysis concentration. Preferably, the Kt/V value is determined by means of an online clearance measurement (OCM) method. For more details of the OCM method, it is referred to DE 3938662 A1. The medical parameters may further comprise a clearance, a body and blood temperature, pulse, EKG and/or EEG data, weight, age and origin of the patient.

Generally, the measurement data may be measured e.g. by means of sensors and/or may be read from a storage (for example if they have been measured before or if they have to be read out from a memory module) and/or may be provided from a prediction module. The different parameters may be received via an interface, in particular the measurement interface, by the CPU and may be processed. In particular, the measurement data may be checked for complying with pre-defined time-related conditions and/or temporal necessities. Further, the measurement data may be checked for correlation or may be evaluated otherwise in order to detect deficient, detrimental or adverse settings of the medical device.

The step aggregating is computer-implemented and/or related to the storage of measurement data. Aggregating may comprise weighting, i.e. assigning priorities, to the different types of measurement data. For example, measurement data, related to the health status of the patient are generally prioritized as high compared to measurement data of less importance. Aggregating may comprise selecting a subset of (e.g. important) measurement data. Aggregating may comprise assigning may comprise meta data and time related data and/or combining specific types of measurement data to a combined dataset. Aggregating may comprise transforming the measurement data in a specific uniform data format for the ease of later processing or may comprise normalizing the measurement data to make later comparison of the different types of measurement data more easily.

The result dataset is prepared to be provided on an output device, for example being a user interface, in particular a graphical user interface (GUI). The output device may be implemented directly on the medical treatment device or may be provided as external device in data communication with the medical treatment device (for example a smart-watch device of the patient). The result dataset is an electronic message. The result dataset may be provided in a textual, graphical, optical (e.g. diode blinking) and/or as acoustic format, for instance as acoustic alert, acoustic signal, or vocalized text.

Countermeasures are actions to be taken in order to mitigate or avoid problematic events before occurrence. Countermeasures may comprise a set of actions and may for example comprise:

- changing treatment parameters, for example in diagnosis ultrafiltrate rates, treatment options, blood flow et cetera.
- Adaptive changes of treatment-prescription aims
- handling existing alarms, alarm signals
- handling predictive alarms
- regular value control checks and their omission it unnecessary, at least unnecessary at that time
- change of dialysate
- adjust concentrate
- addition of anticoagulant, for example heparin
- infusion of medicaments
- pause/stop of treatment
- initiation of re-infusion
- initiation of fluid/blood withdrawal
- other medical interventions aimed at influencing the treatment outcome and/or patient's vital parameters
- machine related manual checkups
- fixing/exchanging deteriorating machine parts or components

The countermeasures may be executed automatically, semi-automatically or manually. The countermeasures may be pre-stored in a storage. This has the advantage that the set of countermeasures may be extended over time and even during the course of application. The countermeasures may be changed for being adapted to new (scientific) developments. Countermeasures may be calculated by means of applying a second machine-learning algorithm in combination with a second trained model. The second model may be trained with training data, comprising aggregated data (or data patterns) and appropriately related potentially problematic events.

As described above, the predicted event occurrences may then be further processed by the CMA algorithm to provide countermeasures that may be calculated on the basis of historical data. The countermeasures are calculated by taking into account temporal necessities for executing the respective countermeasures for the particular problematic event. For example, it is automatically considered, whether a countermeasure may still be applied and executed in due time before the problematic event has been estimated to occur. In case it is estimated that the countermeasure may take longer than the available time left until the occurrence of the problematic event, then, the countermeasure will be denied as unfeasible. Accordingly, automatically, other possible countermeasures which are feasible in the time left are detected. Otherwise, the alarm message is escalated in the result dataset for urgent intervention (in particular if no feasible countermeasures could be detected anymore).

The term "problematic/critical event" or simply "event" should be construed as the occurrence of medical conditions that require automatic, semi-automatic or manual intervention(s), actions and / or measures. Such event occurrences are automatically calculated by the system, presented herein, based on measurement data. Event occurrences may also be predicted by executing a prediction algorithm. This algorithm may be based on historical data. The algorithm for predicting problematic events may be based on a mathematical model. The model may be trained with training data comprising aggregated data and correctly and appropriately assigned events. For example, the aggregated data may represent that the dialysate bag has a volume under a preset threshold, then for this kind of aggregated data (bag volume), the problematic event may be estimated as *"no dialysate available - empty dialysate bag"*. The prediction algorithm may be an adaptive artificial intelligence algorithm which aims to prompt a treatment-related intervention, for instance by a physician, before the critical medical condition effectively occurs. In some situations, in this way the safety of the patient can be increased. Further, the efficiency of the treatment can be increased and critical device conditions, such as software crashes, hardware deterioration, and/or end of disposables etc., may be prevented.

The method makes use of temporal conditions. Monitoring of the system time is therefore important. The time, at which measurement data are received, is usually not the same as the time, at which the result dataset is provided. In particular, the provisioning of the result dataset with the alarm messages may be postponed in case certain conditions are fulfilled. In particular, the system time is the system time of the computer implementing the method of the present invention, e.g. the data-processing system of the present invention. The system time may be measured by a system clock of said computer or an external reference time.

The order, according to which the steps of the method of the present invention are described in the present specification, does not necessarily reflect the chronological order, according to which said steps are carried out.

The accuracy score is a measure of quality for the prediction of a specific event at a specific time. The accuracy score may be represented by a numerical value, an alphanumerical indication (e.g. as a traffic light signal, representing "green" for sufficient accuracy and "red" for insufficient accuracy). Further, the accuracy score may be provided as a percentage (compared to 100% accuracy). The accuracy score is typically affected by the predicted condition and the predicted time; e.g. for alarm "critical blood pressure in 1h" with predicted blood pressure to be at critical value (+/- 20%) in 1h (+/- 20%) an accuracy score of e. g. 80% could be issued. Depending on the accuracy score (above/below a predefined value), the algorithm may decide to initiate further measures or to wait for new measurement data in order to improve the accuracy score.

The reliability indicator is a measure for the predicted effect of a countermeasure on the (prevention of the) predicted condition. The reliability indicator may be represented by a numerical value. The reliability indicator is usually affected by match/overlap of characteristics between currently predicted condition and historical conditions, historical success rate of countermeasures to prevent the condition, and relation of estimated countermeasure duration to remaining time until occurrence; e.g. for handling alarm "critical blood pressure in 1h": currently measured blood pressure pattern matches to 60% with a blood-pressure pattern from historical data patterns preceding occurrence of critical values; most appropriate historical countermeasure prevented the event in 60% of the cases; countermeasure has never been performed in the current environment, but shows 80% characteristics of a comparable countermeasure available, the countermeasure is estimated to take 1h (+/-20%) of time. Here, a reliability score of e. g. 70% could be issued.

The accuracy score relates to the prediction of problematic events and defines, with respect to preset thresholds, when the analysis for a potential countermeasure (or other action) is considered. The reliability indicator relates to the prediction of countermeasures and defines the "if - when" and content of a user prompt. The accuracy score and the reliability indicator are both issued to make the algorithm's "decision" transparent and understandable to the user.

The properties, features and advantages of this invention described above, as well as the manner they are achieved, become clearer and more understandable in the light of the following description and embodiments, which will be described in more detail in the context of the drawings. This following description does not limit the invention on the contained embodiments. Same components or parts can be labeled with the same reference signs in different figures. In general, the figures are not to scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

## BRIEF DESCRIPTION OF THE DRAWINGS

Fig. 1 is a flow chart of a method according to a preferred embodiment of the present invention;

Fig. 2 is a schematic figure of the structure of a result dataset according to a preferred embodiment of the present invention;

Fig. 3 is a representation of a sequence of processed datasets according to a preferred embodiment of the present invention;

Fig. 4 is another representation of a sequence of processed datasets according to an alternative embodiment, in relation to the one shown in figure 3;

Fig.5 is a schematic representation for all the reception of measurement data from different sensors, devices, apparatuses or from prediction module according to a preferred embodiment of the present invention;

Fig.6 is an overview figure showing the countermeasure algorithm in more detail;

Fig. 7 is a schematic representation of a medical device, for example dialysis machine with an alarm handler in different example embodiments;

Fig. 8 represents a schematic flow chart of processes to be executed on a medical treatment device and/or on a remote server.

## DETAILED DESCRIPTION OF EMBODIMENTS

The present invention relates to a temporally controlled alarm handling for a medical device, in particular for a dialysis machine. The present invention relates to methods, systems, electronic modules and apparatuses as well as products for determining a set of alarm messages to be output on, for example, a graphical user interface of a medical device, like a dialysis machine.

The present invention provides a solution for the fact that in medical appliances with medical treatment machines the timing is a critical parameter. Although it is known to process a huge variety of measurement data, it is still an unmet need to process certain subsets of the total of all measurement data for which the predicted impact on the safety of the medical treatment by means of the medical treatment device operation is high. This may be achieved by evaluating these measured data, for instance by evaluating a factor-weighted impact on the occurrence of certain critical conditions and a prediction that directly influences the effect of the device and, thus, the outcome of the medical treatment.

The solution described herein directly interfaces with the user and thus improves user interventions on the medical treatment device.

As mentioned above, the timing is a critical aspect. On the one hand it would be helpful to evaluate those measurement data, which are taken temporarily as close as possible to the time of the predicted occurrence of critical events, because these measurements being close to the problematic event appearance generally show a high accuracy score. On the other hand, the calculated or determined countermeasure [which has been provided by the system] also takes a certain amount of time to be executed in order to influence (mitigate) the occurrence of the critical condition, which supports to evaluate the measurement data as early as possible and not as close as possible to the problematic event occurrence. This contradicting use of time as a resource is considered in the present invention by a modified provisioning of alarm messages for operating the medical device. The advantages may be summarized as follows:

- the prediction accuracy of predicted, estimated or calculated data is increased.
- It is ensured that "enough" time is remaining for the interventive measure to positively affect the devices operation and/or to prevent the problematic state or condition.
- The stress on the medical treatment device, the staff, and/or the patient is reduced.
- The consumption of disposables/consumables is reduced, too.

In particular, relating to safeguarding that sufficient time remains for execution of the countermeasure, it is necessary to evaluate and process measurement data beyond the patient data and device data, and to also use external data, relating to the clinical infrastructure, to locations of users and devices, user experience, allocation of disposables, etc.

In a preferred embodiment the received measurement data are compared in their characteristic patterns with a development over time (temporal development of these patterns) to prestored reference patterns. The prestored reference patterns may be provided as results of artificial-intelligence algorithms, which have been trained with previously obtained and analyzed data of the same, or of comparable, kind. In case of a match between the received measurement patterns and the reference patterns, which may for example be stored in an analytical database, an alarm message is determined to be executed (provided) in order to prevent the forecasted critical condition to occur. In a further embodiment, an accuracy score is provided. The accuracy score represents the accuracy of the calculation and may be conveyed to the user on a user interface in order to represent a reliability of the prediction. Based on the provided results data set with a set of alarm messages, a browser element might be provided on a user interface for giving the user the opportunity, to trigger an additional analysis (e.g. further statistical evaluation).

One key aspect of the solution described herein is to process estimated durations of tasks or actions, counteracting the development of the underlying measurement data in case problematic event occurrences are predicted based upon these very measurement data. The processing is based on a set of algorithms.

In a preferred embodiment, the method and related apparatus (alarm handler, device and computer program) consider the accuracy score of the prediction as distinction between two different sub-processes, namely:

- to (already) use the actual measurement data for prediction, namely for determining the set of alarm messages, although the measurement data are not close to the time of the predicted event occurrence or
- to not use the actual measurement data for prediction (determination of alarm messages), but instead to wait for future measurement data in order to evaluate the temporal development of the received measurement data in order to improve and enhance the accuracy score for the prediction.

This feature has the technical advantage that the accuracy score of the provided predictions (i.e. the calculations and determinations for providing the result dataset) serves as a basis for an algorithmic decision whether to rely upon the actual measurement data or better to postpone the provisioning of the result dataset (by prompting the user to initiate countermeasures) and to wait for new data in order to process the development of the measurement data over time in order to improve the accuracy of the prediction. This decision is taken in view of the estimated duration of the countermeasure. In particular, the decision to wait for new measurement data can only be applied if sufficient time is left for executing the counter measure.

The estimation of the time duration of the determined countermeasure is based on an identification of a set of characteristics of the respective countermeasure, of historical countermeasures, which have been applied, and their durations, serving as reference measures. Thus, the similarity (matching score) may be calculated between the reference measures and the determined countermeasure. A measure for the accuracy of the prediction may be calculated, for instance a standard variation for normally distributed durations as part of a reliability score.

The determined duration and scores can be transmitted to the user interface for being displayed. The user may then evaluate the presented data and trigger the countermeasures, or the user may initiate further/additional analysis, or the user may decide on further actions by for instance alarming, ignoring, observing, passively triggering automatic measures and actions.

In case no best match between measurement data (pattern) and reference data (pattern) exist, benchmark data may be applied based on similar measures having a similar but not identical pattern. For example, if the countermeasure relates to the handling of a sudden blood pressure variation, and no identical pattern is available, then a benchmark data set may be applied, which relates to the appropriate countermeasure which has been found in another clinic or institution for that situation.

The patterns of measurement data may be generated by considering relevant medical treatment devices and its equipment, disposables, mechanical components etc., relevant personnel, relevant skill sets, relevant measurement data, relevant location data and/or navigation-related data, prior duration estimates and scores, relevant drugs, their combinations and their availability, physiological effects and risks, and others. All these characteristics or a subset thereof are evaluated based on both quantitative and qualitative properties. The characteristics can be correlated in different combinations and certain combinations can be used to structure meta characteristics, representing the effort for a given staff to perform or execute a countermeasure.

It is to be noted, that in the following detailed description, letter-based reference signs (for instance *am, md, rd* etc.) are formatted in italic for the ease of textual understanding; whereas, in the claims, these reference signs are formatted in normal font (i.e. non-italic).

**Figure 1** shows a sequence of method steps according to a preferred embodiment of the invention. After start of the system, in step S1 measurement data *md* are received from a plurality of sensors, data providers, and/or from a prediction module(s). The measurement data *md* may comprise technical parameters and health-related parameters from the patient or medical values as well as values output from a prediction module, for example relating to the lifetime of our technical component of the medical device or a related set of apparatus. Also alarm signals of components of the medical device may serve as measurement data. In contrast to prior systems according to the state of the art, the alarm signals are no longer directly output by the system but are now being processed in order to provide an alarm message (in processed form) which is intelligently constructed and thus more sophisticated and more adapted to the specific use case as will be described in more detail below.

In step S2, the received measurement data *md* are aggregated. The aggregating step may comprise storing the received measurement data on a local or central storage (memory). In a preferred embodiment the step of aggregating may comprise selecting measurement data, combining measurement data, normalizing, and/or transferring measurement data.

In step S3, the aggregated data are processed on the computer or central processing unit thereof by applying the temporal alarm significance algorithm, also abbreviated as TASA. The TASA serves to provide a result dataset rd with a determined set of alarm messages and a calculated execution time t, defining when/at what time the determined set of alarm messages is going to be executed or provided on a user interface.

The method described in this disclosure has the technical advantage that the medical treatment may be executed more efficiently and safely. The user (e.g. medical staff) is no longer distracted by a plurality of unnecessary alerts or alarm signals which are not urgent or do not need urgent intervention. Contrariwise, the result dataset only comprises such alarm messages which correspond to the real need for prompt interaction and intervention. In particular, if an intervention may be postponed, the respective alarm message will either not be issued at that time (will only be issued in a later phase) or be displayed with corresponding metadata, representing that the intervention may also be executed later. Thus, the user may concentrate on urgent interventions and is no longer distracted by distracting notifications.

**Figure 2** is a schematic representation of a result dataset *rd* according to several example embodiments. The result dataset *rd* comprises a set of alarm messages *am* and a calculated execution time *et.* The execution time *et* represents a trigger signal for triggering display or alternate provisioning of the respective alarm message *am,* i.e. when the alarm message is e.g. to be displayed on the user interface UI. The calculation of the execution time *et* is based on the temporal indication *ti* received from the measurement data *md* and is further based on the determined duration of the respective countermeasure cm. As can be seen in figure 2, the alarm message *am* comprises the countermeasure, which has been analyzed to be available as temporally feasible and necessary to prevent or mitigate the predicted event prior to its predicted occurrence by taking into account the estimated availability and duration of the countermeasure. This is represented in the figure 2 with the reference numeral $cm_{eo-t}^{a|d}$.

In general, temporal developments are processed for the measurement data *md,* for the execution or provisioning of the alarm message *am,* the duration of the countermeasure cm.

As can be seen in figure 2, the result dataset *rd* may further comprise the predicted (problematic) event occurrence *eo.* This helps the user getting more specific information about the future risks of the medical treatment, which are due to the actual treatment status, being represented by the received measurement data *md.* Further, the result dataset *rd* may additionally comprise a reliability indicator *ri,* which represents by value how reliable the prediction/estimation which has been calculated, is. Further, the result dataset *rd* may comprise an accuracy score as, which represents by a value how accurate the prediction/estimation which has been calculated, is. Further, the result dataset *rd* may comprise metadata relating to the actual status of the medical treatments device or the medical treatment process. For example, the metadata may comprise a sequence of actions to be taken in order to mitigate or avoid the problematic event.

**Figure 3** shows a sequence of processed data according to a preferred embodiment of the present invention. The received measurement data *md* together with the temporal indication *ti* are transferred into aggregated data *ag,* which again are processed in an event occurrence with time indication eo-t. The latter step may be executed by a prediction algorithm, which has been described above, with respect to the step of predicting S31. The event occurrence with time indication eo-t is then processed to the countermeasures, for which the estimated availability and duration is taken into account in view of the event occurrence with time indication, which is represented in the figures by reference numeral $cm_{eo-t}^{a|d}$. The latter step may be executed by means of applying a determination algorithm, which has been mentioned above, with reference numeral S32. The calculated countermeasures $cm_{eo-t}^{a|d}$ may be further processed in order to provide the result dataset *rd*.

**Figure 4** shows an alternate embodiment of the sequence of data processing compared to the one shown in figure 3. As can be seen, the measurement data together with the temporal indication (*md* + ti) serves as a basis for aggregating data *ag.* The aggregated data *ag* may be processed by an alternate determination algorithm S 31' in order to provide at least one event occurrence *eo*. As can be seen in figure 4, aggregated data *ag* serve as a basis for providing time indication t, too. The event occurrence *eo* may be further processed by an alternate determination algorithm S32' for determining the countermeasures $cm_{eo}^{a|d}$, in this case without time indication t. The time indication t is then processed to provide the final countermeasures for which the estimated availability and duration is taken into account in view of the estimated availability and duration of the countermeasure $cm_{eo-t}^{a|d}$. by processing the time indication t provided with the aggregated data ag, which then maybe processed to provide the final result dataset *rd*.

**Figure 5** shows a schematic representation of different embodiments for the reception of the different types of measurement data *md.* As can be seen in figure 5, the measurement data *md* may stem from a plurality of different sensors or data providers. For example, the measurement data *md* may be received from a smart watch (providing physiological data), from the medical treatment device MD, in particular from a dialysis machine, from medical sensors, for example a blood-saturation sensor, which is applied to the fingertips of the patient, or from a prediction module PM or from other technical devices or components. In this respect it has to be noted that all measurement data are combined with temporal indication, indicating when the signal has been measured, for instance as a time stamp. The processing of the time indication t is necessary in order to evaluate possible countermeasures whether or not they are feasible within the time left before the problematic event possibly may occur. As represented in figure 5, the prediction module PM may process different types of measurement data *md* in order to predict related safety-critical events. For example, if the blood pressure has been measured as exceeding a preset threshold and in addition if the temperature of the patient has been measured to be above a preset threshold, the prediction module PM may predict a physiological problem of the patient, so that related measurement data may indicate an inflammatory process, which need instant intervention, which will be represented in the result dataset rd, correspondingly. Another example relates to e.g. the combination of a (constant) rotation frequency of a device pump with a downward trend from a down-stream fluid-pressure sensor. This might indicate a fluid-line leak that requires technical intervention.

**Figure 6** shows a schematic representation of the countermeasure algorithm CMA. Depending on the preferred embodiment, event occurrences without or with time indication *eo, eo-t* are provided to and received from the CMA in order to execute the algorithm for determining at least one countermeasure S 32, S 32' for providing the respective processed data sets with countermeasures $cm_{eo-t}^{a|d}$ $cm_{eo}^{a|d}$.

The CMA algorithm may access a first machine-learning tool with a first set of machine learning algorithms ML1, which are based on a first trained model TM1.

As can be seen in Fig. 6, the prediction of safety-critical events eo (or their occurrences) may also be based on a second machine-learning tool with a second set of machine learning algorithms ML2, which are based on a second trained model TM2.

**Figure 7** shows a simplified schematic representation of a medical device MD, such as a dialysis machine. The medical device MD is equipped with a measurement interface MI, which is configured for receiving the measurement data *md.* The medical device MD further comprises a storage S, which is in data connection with a central processing unit CPU, which is configured for having implemented thereon the TASA and optionally the CMA. In another preferred embodiment the CMA may also be implemented on another unit, for instance on a graphical processing unit GPU (not shown) or on an external unit, not included on the medical device MD.

The medical device MD preferably comprises a secondary storage 2S with a second trained model TM2 for execution of the (second) machine learning algorithm ML. The secondary storage 2S may be combined with the storage, previously mentioned. Upon execution of the TASA algorithm, the result dataset *rd* is provided. The result dataset rd may be prepared and triggered for output on a user interface UI, which preferably may be implemented on the medical device MD. However, as depicted in figure 7 in dotted lines, the user interface UI may also be implemented on a device external apparatus, for instance smart device, assigned and related to the nurse. As well as the CPU, the secondary storage may also be implemented on an external unit, including a set of distributed units, and connected to at least one medical device MD by means of data connection.

**Figure 8** represents a sequence of processes and steps to be executed on the medical treatment device and/or on the remote server, which may be implemented as cloud server, being accessible over respective network interfaces. As can be seen, based on the received measurement data, it is evaluated whether a critical event may occur or not and, if yes, the problematic event occurrence may be displayed on the user interface. In order to predict event occurrences, identified data patterns are compared to reference patterns, for example of historical data. In case a problematic event is predicted, the countermeasure algorithm is executed for identifying suitable countermeasures which are executable and feasible within the time left until the predicted event occurrence.

Relating to figure 8, preferably the following steps are executed on a user interface by way of user interaction:

- Ignore output/ passively trigger automatic further analysis or actively initiate further analysis;
- Initiate countermeasures;
- Manual user feedback.

The following steps are preferably executed on the medical treatment device MD:

- The five first steps, which are depicted in the left column ("generate data form sensor measurements" until "Prompt action/input considering still feasible actions");
- On-line data gathering.

The following steps are preferably executed on a - e.g. cloud-based - server, which is in network connection to the medical treatment device MD:

- All the steps, which are depicted in the second column, besides the first one (starting with "identify data patterns");
- Estimate duration of countermeasures;
- Evaluate effect/duration of performed process;
- Identify task characteristic;
- Compare present task to historical task;

The rest of the steps, depicted in figure 8, may preferably be executed by means of database access or directly on a database.

In the following, four example applications will be described in more detail.

1) The patient's blood pressure is measured continuously as one type of measurement data *md.* In system time T1 a development towards critical values is predicted, with a critical value occurring in 20 minutes (with respect to T1). The staff's intervention procedure, i.e. the countermeasure *cm*, is estimated to take three minutes between alarming and the effect, i.e. prevention of occurrence of critical blood-pressure values, to take place. The alarming may be implemented as providing the result dataset *rd*. The alarming is automatically postponed for 10 more minutes. In this time, the patient's blood pressure is continuously monitored and is determined to change towards normal level again. Accordingly, a respective result dataset *rd* is provided without any alarm issuance. In a preferred embodiment, the result dataset *rd,* provided at system time T1 may comprise the predicted event occurrence with the reliability indicator ri, which, in this case, indicates that the reliability is very low and that therefore blood pressure development is further monitored. The result dataset *rd* may comprise the measurement values as well.

2) The volume of the dialysate bag is measured continuously and received as measurement data *md.* It is predicted to be depleted in 1 hour. Based on historical data, the change of the bag by the staff takes place within 10 minutes after notification. 10 minutes before the bag is predicted to be empty, the staff is notified to prepare countermeasures *cm.* The treatment pause due to bag change is reduced. In another dialysis clinic, the staff may be less trained, and the clinic may be less organized with longer walking distances to get a new bag. In this case, the bag change is predicted to take 30 minutes. Accordingly, staff notifications (providing the result dataset *rd* with the countermeasures *cm*) therefore are issued earlier in advance in this clinic.

3) In another example, a certain patient usually has a variety of occurring problems during the treatment. Based on the measurement data *md* of his vitals, one of those problems can be predicted to occur in the next 20 minutes. However, the prediction is very inaccurate and not reliable. The staff has set the device to a reliable prediction mode to not be bothered by false alarms. Thus, no alarm is issued yet. 10 minutes after the first prediction, the prediction accuracy is determined to be increased (accuracy score). Now, it is quite certain, that the problem will occur in 10 minutes. The staff is notified accordingly with an appropriate alarm message *am.* The staff, thereupon, performs a countermeasure cm to solve the problem before it happens. Alternatively, when arriving, the staff is presented with different intervention processes or countermeasures *cm* and their estimated durations. The staff decides that one suggested countermeasure *cm* that is estimated to take one minute to perform, will be enough in case the prediction is correct. However, the staff does not trust the prediction yet and waits for five more minutes, after which the prediction becomes 100% certain. The list of suggested countermeasures *cm* is now limited due to the remaining estimated time, but the previously preferred countermeasure is still suggested. The staff performs a countermeasure cm to solve the problem before it happens.

4) In still another example a mechanical part of the medical treatment device is supposed to function one year before it needs to be replaced. Further, suppose, that in the country, where the medical device is used, technicians are very busy. It is estimated that the machine part replacement takes half a year after notification (result dataset *rd* with alarm message *am,* in this case: "replacement notification") of the technician. The technician is notified well in advance by the result dataset *rd* to make sure that he/she replaces the failing part in time. No downtime due to broken part occurs.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

A single unit or device may fulfil the functions of several items recited in the claims. The mere fact that certain measures are recited in mutually different dependent claims does not indicate that a combination of these measures cannot be used to advantage.

Moreover, those skilled in the art will appreciate that at least some aspects of the present invention may be practiced in other configurations, including hand-held and/or mobile devices, multiprocessor systems, microprocessor-based or programmable consumer electronics, embedded systems, network computers, minicomputers, set top boxes, mainframe computers, and the like. At least some aspects of the present invention may also be practiced in distributed computing environments, as mentioned above, where tasks are performed by remote processing devices linked through a communications network. In a distributed computing environment, program modules may be located in local and/or remote memory storage devices.

Any reference signs in the claims should not be construed as limiting the scope.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

## Claims

**1.** Computer-implemented method for determining a set of alarm messages (am) for operating a medical device (MD), comprising the method steps of:

- Receiving (S1) measurement data (md) with a temporal indication (ti) from a set of sensors and/or data providers;
- Aggregating (S2) the received measurement data (md);
- Processing (S3) the aggregated data (ag) by applying a temporal alarm significance algorithm (TASA) for providing a result dataset (rd) with a determined set of alarm messages (am) and a calculated execution time, defining when the determined set of alarm messages (am) are to be executed.

**2.** Method according to claim 1, wherein the temporal alarm significance algorithm (TASA) comprises:

- Based on the aggregated data (ag): Predicting (S31) at least one event occurrence with temporal indication (eo-t,eo, t);
- Determining (S32) at least one countermeasure (cm) for the predicted event occurrence (eo) and its estimated availability and duration;
- Determining (S33) the set of alarm messages (am) with countermeasures (cm) which have been analyzed to be available and temporal feasible and necessary to prevent or mitigate the predicted event prior to its predicted occurrence by taking into account the estimated availability and duration of the countermeasure (cm).

**3.** Method according to the directly preceding claim, wherein analyzing the availability of a countermeasure (cm) is based on device-external data and may in particular be based on a clinical infrastructure parameter, a location-based parameter of patient and/or devices and/or staff for executing the countermeasures (cm), a user-experience parameter, an allocation parameter, representing allocations of disposables and/or equipment for countermeasure execution.

**4.** Method according to any of the preceding claims, wherein the method comprises:

- Providing the determined set of alarm messages (am) on an output interface (UI) at the determined execution time.

**5.** Method according to any of the preceding claims, wherein the measurement data (md) are received from a medical treatment device (MD), a patient's device and/or equipment, a data provider, and/or a prediction module (PM).

**6.** Method according to any of the preceding claims, wherein the measurement data (md) comprise original alarm signals, issued by a medical treatment device (MD) and wherein the determined set of alarm messages (am) is a subset or a superset of the original alarm signals.

**7.** Method according to any of the preceding claims 2 to 6, wherein countermeasures (cm) are determined by executing a countermeasure algorithm (CMA), which may be based on a machine-learning algorithm (ML) which has been trained in a supervised or semi-supervised manner with training data of historical problematic events and associated countermeasures (cm).

**8.** Method according to any of the preceding claims, wherein the execution time for the determined set of alarm messages (am) is calculated by processing estimated process durations of countermeasures (cm).

**9.** Method according to the directly preceding claim, wherein process durations for execution of the countermeasures (cm) are estimated by accessing a storage with a countermeasure data structure in which each countermeasure (cm) is assigned a unique identifier and corresponding characteristic properties, including process duration so that process durations may be determined for processes which have similar characteristic properties.

**10.** Method according to any of the preceding claims, wherein the determined set of alarm message (am) and/or the result dataset (rd) include meta data (meta), comprising operating instructions on a step-by-step basis.

**11.** Method according to any of the preceding claims, wherein the determined set of alarm messages (am) and/or the result dataset (rd) comprise/s a reliability indicator (ri), defining the accuracy and/or reliability of predicted data, in particular of events (eo) and countermeasures (cm) and/or wherein the temporal alarm significance algorithm (TASA) processes the reliability indicator (ri).

**12.** Alarm handler (AH) for determining a set of alarm messages (am) for operating a medical device (MD), configured to perform a method according to any of the preceding method claims, comprising:

- A measurement interface (MI) which is adapted for receiving (S1) measurement data (md) with a temporal indication from a set of sensors and/or data providers;
- A storage (S) for aggregating (S2) the received measurement data (md); and
- A processing unit (CPU) for processing the aggregated data (ag) by applying a temporal alarm significance algorithm (TASA) for providing a result dataset (rd) with a determined set of alarm messages (am) and a calculated execution time, defining when the determined set of alarm messages (am) are to be executed.

**13.** A medical device (MD) with an output interface (UI) and further comprising an alarm handler (AH) according to the directly preceding claim.

**14.** A computer-implemented system for determining a set of alarm messages (am), comprising:

- An alarm handler (AH) according to claim 12;
- A medical device (MD) and
- An output interface (UI) for providing the result dataset (rd).

**15.** A computer program comprising a computer program code, the computer program code when executed by a processor causing a medical device or an alarm handler or a processing unit to perform the steps of the method of any of the preceding method claims.

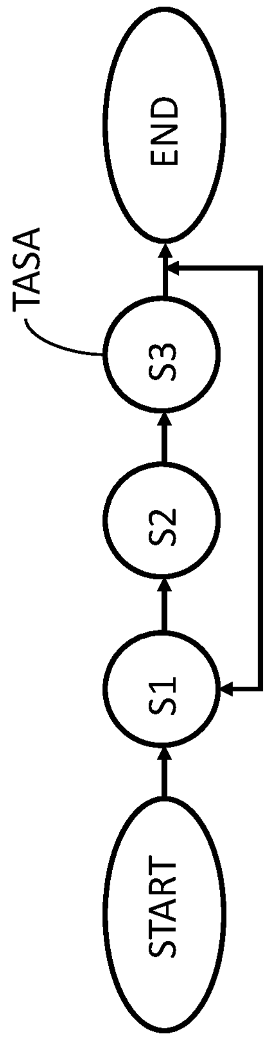


FIG. 1

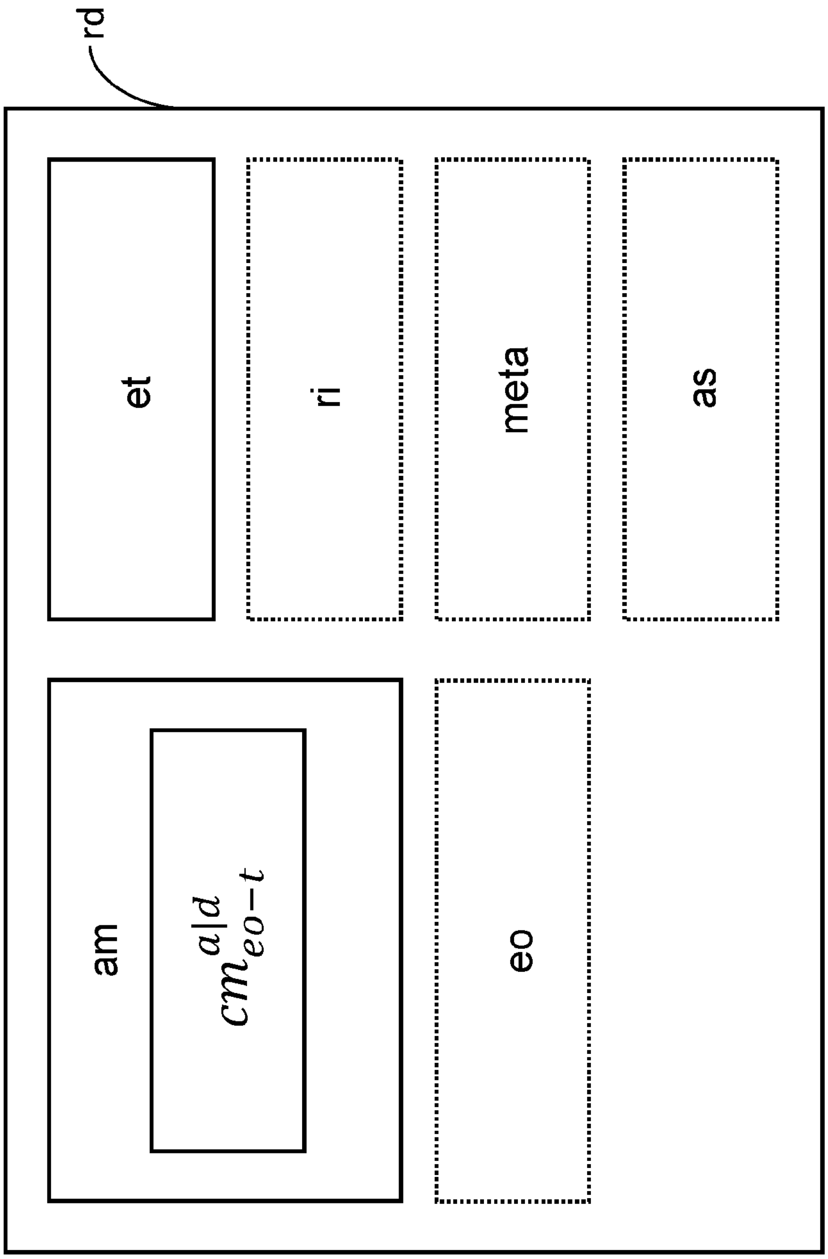


FIG. 2

FIG. 3

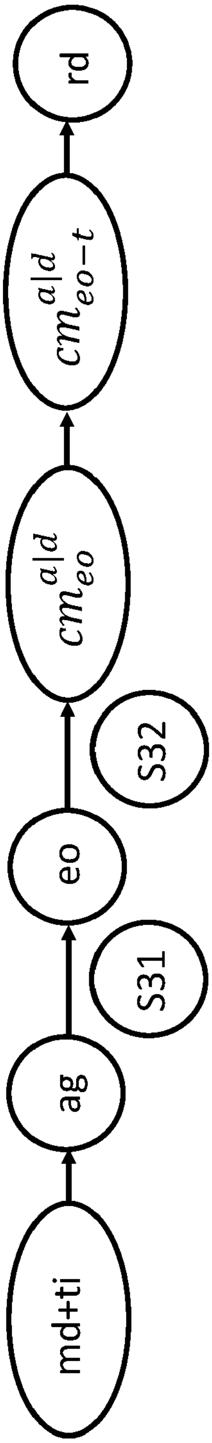

FIG. 4

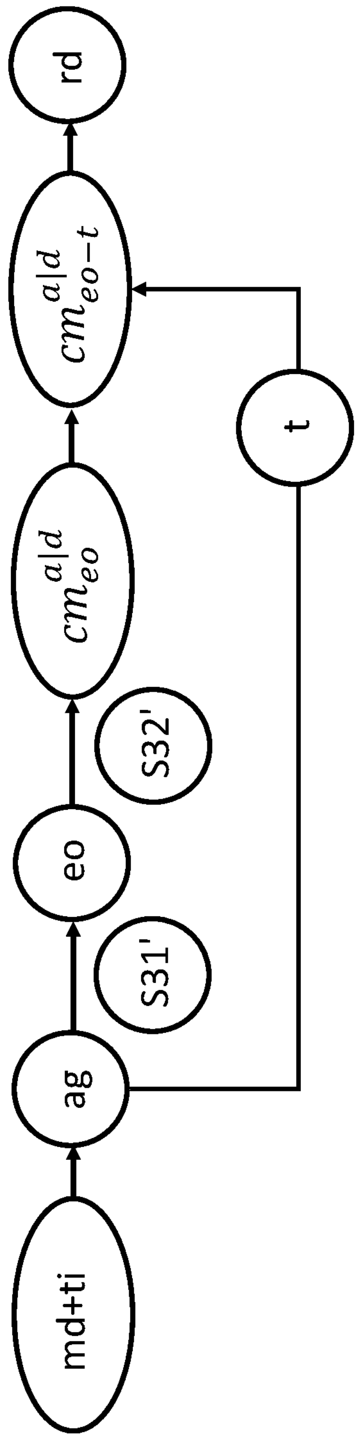

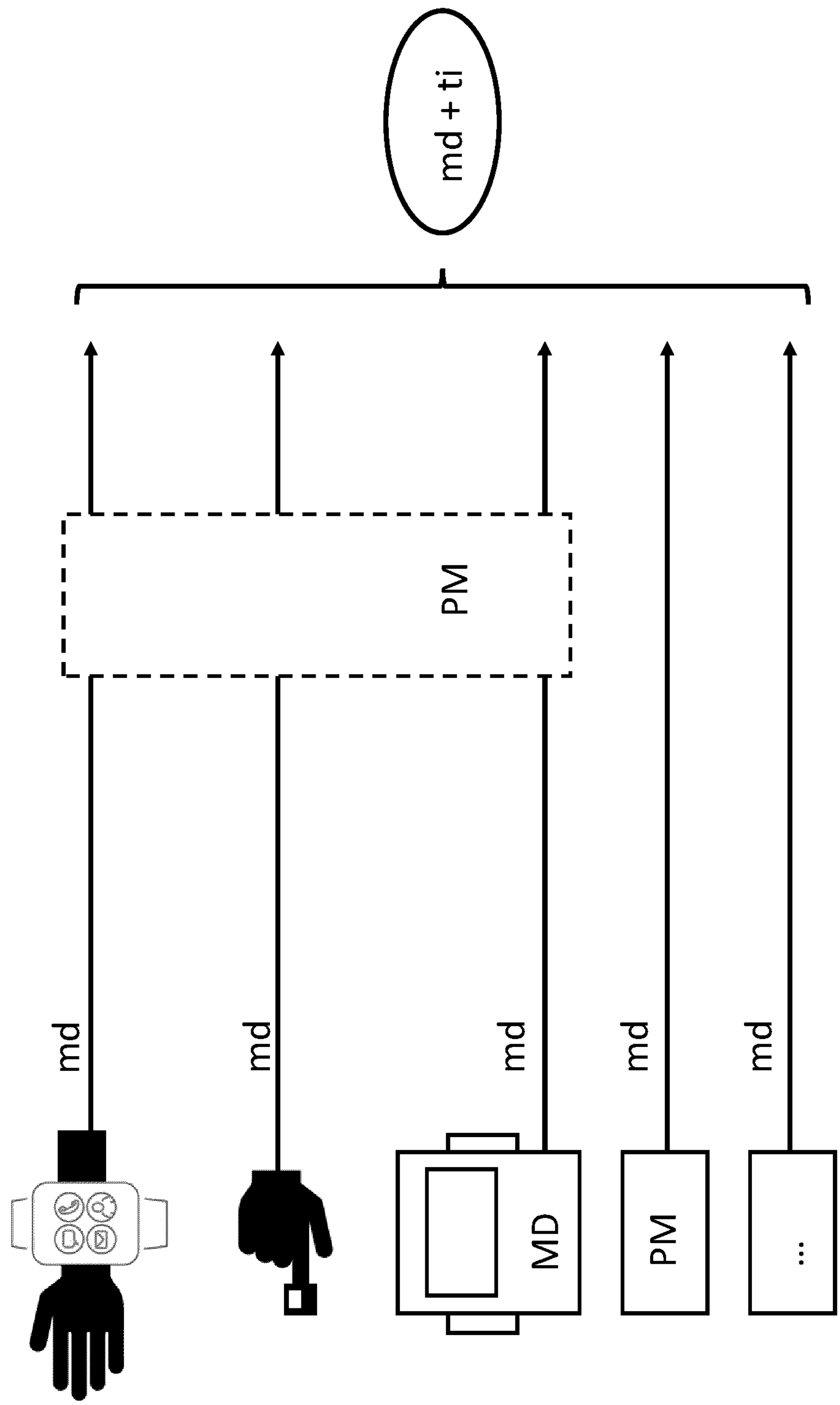


FIG. 5

FIG. 6

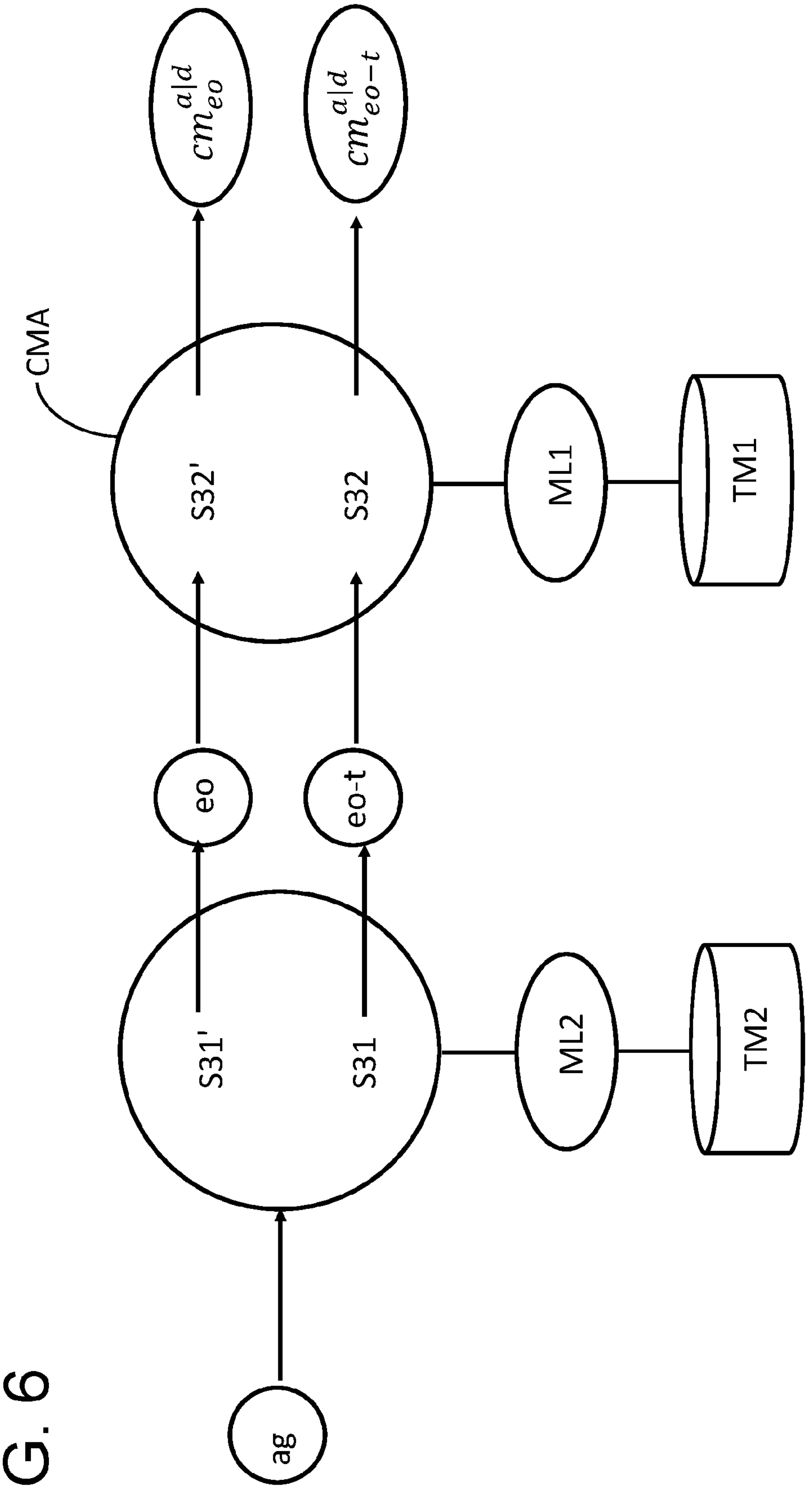

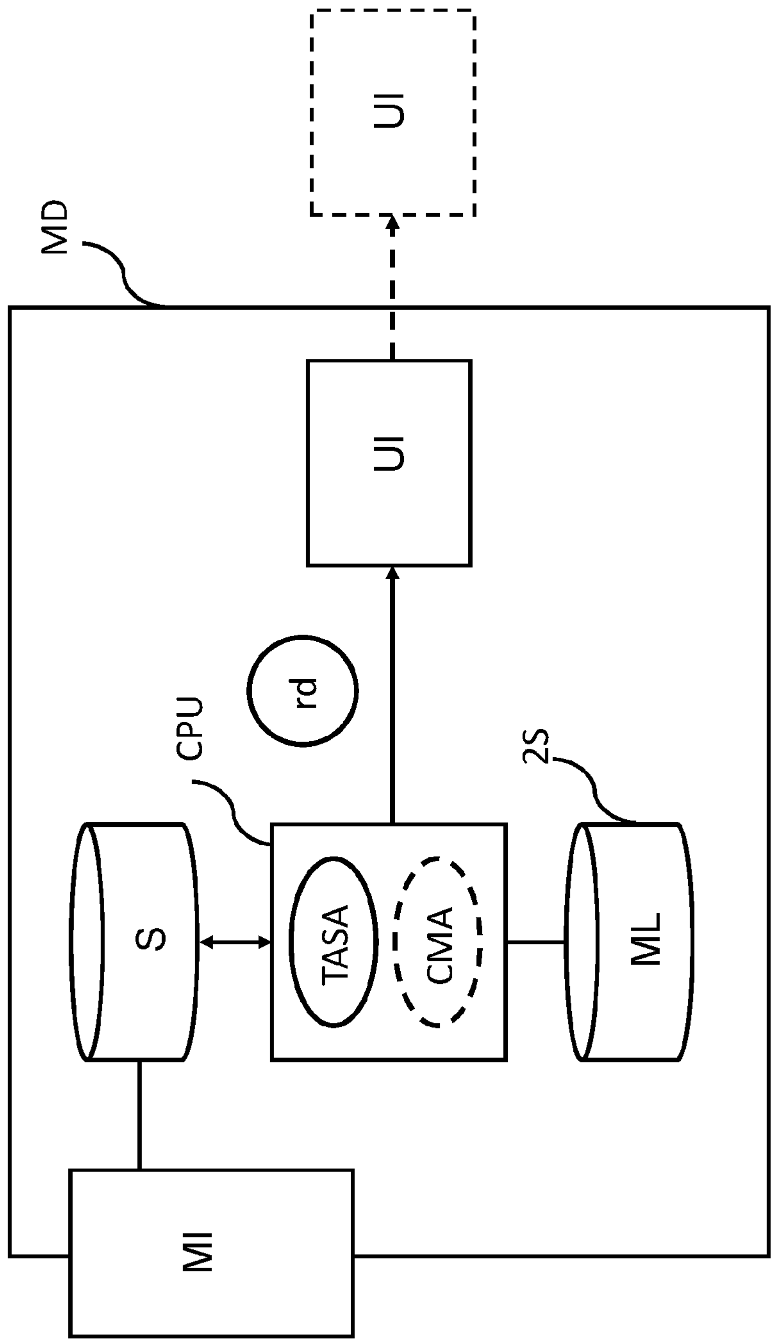


FIG. 7

FIG. 8

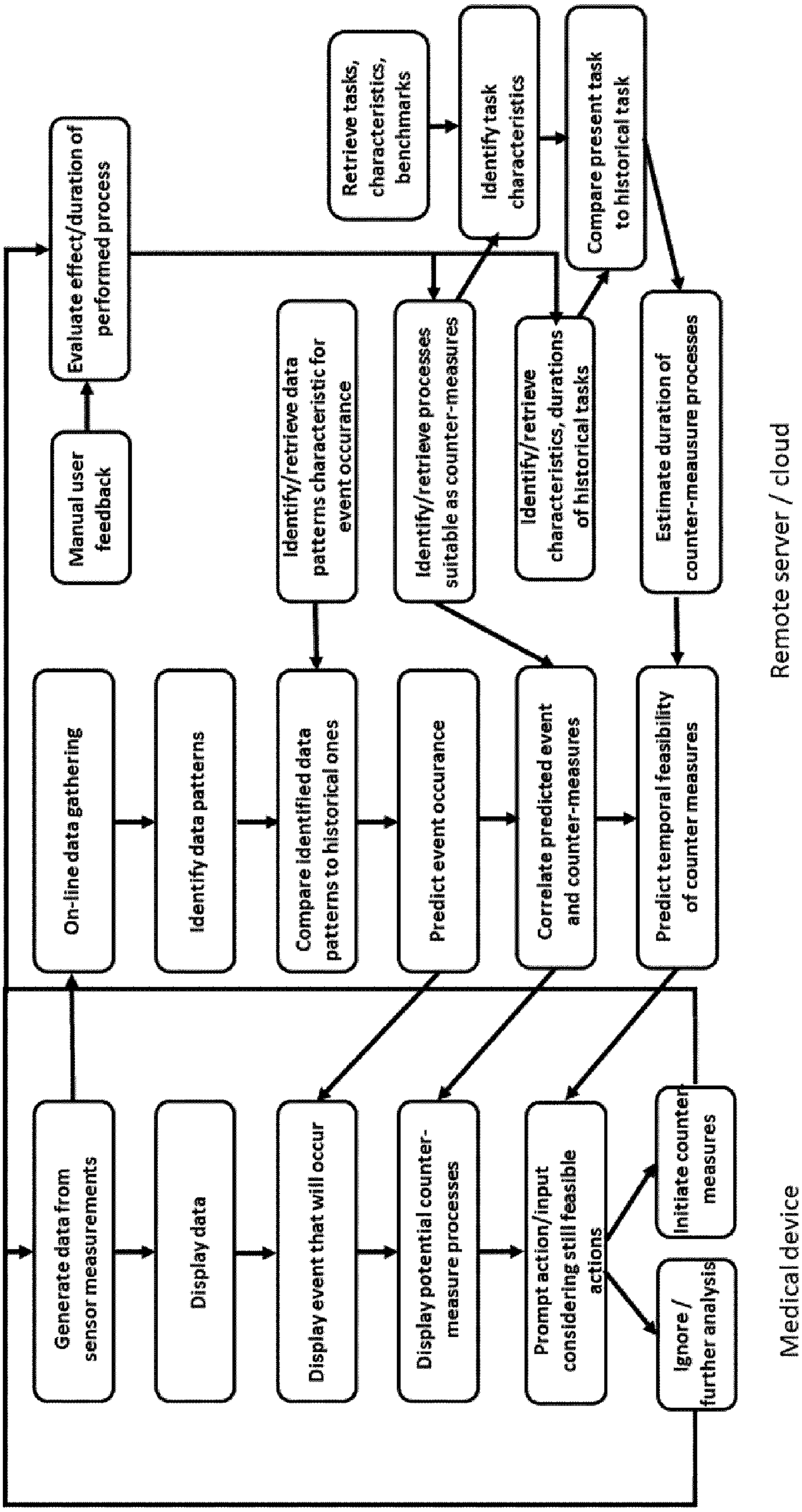
Generate data from sensor measurements
Display data
Display event that will occur
Display potential counter-measure processes
Prompt action/input considering still feasible actions
Ignore / further analysis
Initiate counter measures
On-line data gathering
Identify data patterns
Compare identified data patterns to historical ones
Predict event occurance
Correlate predicted event and counter-measures
Predict temporal feasibility of counter measures
Manual user feedback
Evaluate effect/duration of performed process
Identify/retrieve data patterns characteristic for event occurance
Identify/retrieve processes suitable as counter-measures
Identify/retrieve characteristics, durations of historical tasks
Estimate duration of counter-meausure processes
Retrieve tasks, characteristics, benchmarks
Identify task characteristics
Compare present task to historical task
Remote server / cloud
Medical device

Europäisches Patentamt
European Patent Office
Office européen des brevets

# EUROPEAN SEARCH REPORT

Application Number
EP 20 46 5536

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 01/21067 A1 (UNIV FLORIDA [US]; IASEMIDIS LEONIDAS D [US] ET AL.) 29 March 2001 (2001-03-29)<br>* pages 3,6,7,25; claims; figures * | 1,12-15<br><br><br>1-15 | INV.<br>G16H40/40<br>A61B5/00<br>G16H40/60<br>G08B21/00<br>G16H20/17 |
| Y | WO 2010/023914 A1 (JMS CO LTD [JP]; TAKEBAYASHI MASAAKI [JP] ET AL.) 4 March 2010 (2010-03-04)<br>* paragraph [0012]; claims; figures 25,26 * | 1-15 | |
| X<br><br>Y | US 5 135 485 A (COHEN LOUIS [US] ET AL) 4 August 1992 (1992-08-04)<br>* Col. 2,8,10,11; claims; figures * | 1,12-15<br><br>1-15 | |
| X<br><br>Y | WO 2014/202445 A1 (KONINKL PHILIPS NV [NL]) 24 December 2014 (2014-12-24)<br>* pages 6,7; claims; figures * | 1,12-15<br><br>1-15 | |
| X<br><br>Y | US 2020/111568 A1 (BISHOP COLBY [US] ET AL) 9 April 2020 (2020-04-09)<br>* paragraphs [0051] - [0054], [0059]; claims; figures * | 1,12-15<br><br>1-15 | TECHNICAL FIELDS SEARCHED (IPC)<br>G16H<br>A61B<br>G08B |

The present search report has been drawn up for all claims

1

| Place of search | Date of completion of the search | Examiner |
|---|---|---|
| Munich | 9 November 2020 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 46 5536

This annex lists the patent family members relating to the patent documents cited in the above-mentioned European search report. The members are as contained in the European Patent Office EDP file on
The European Patent Office is in no way liable for these particulars which are merely given for the purpose of information.

09-11-2020

| Patent document cited in search report | Publication date | Patent family member(s) | Publication date |
|---|---|---|---|
| WO 0121067 A1 | 29-03-2001 | AT 447358 T | 15-11-2009 |
| | | AU 767939 B2 | 27-11-2003 |
| | | CA 2383218 A1 | 29-03-2001 |
| | | EP 1225832 A1 | 31-07-2002 |
| | | JP 4910095 B2 | 04-04-2012 |
| | | JP 2003509149 A | 11-03-2003 |
| | | US 6304775 B1 | 16-10-2001 |
| | | WO 0121067 A1 | 29-03-2001 |
| WO 2010023914 A1 | 04-03-2010 | BR PI0912931 A2 | 24-09-2019 |
| | | CN 102131535 A | 20-07-2011 |
| | | JP 2010051720 A | 11-03-2010 |
| | | KR 20110055624 A | 25-05-2011 |
| | | WO 2010023914 A1 | 04-03-2010 |
| US 5135485 A | 04-08-1992 | NONE | |
| WO 2014202445 A1 | 24-12-2014 | CN 105324069 A | 10-02-2016 |
| | | EP 3010398 A1 | 27-04-2016 |
| | | JP 6199486 B2 | 20-09-2017 |
| | | JP 2016526405 A | 05-09-2016 |
| | | US 2016113595 A1 | 28-04-2016 |
| | | WO 2014202445 A1 | 24-12-2014 |
| US 2020111568 A1 | 09-04-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 3938662 A1